# EUROPÄISCHE PATENTSCHRIFT

(11) **EP 2 070 506 B1**
(45) Veröffentlichungstag und Bekanntmachung des Hinweises auf die Patenterteilung: **24.04.2013**
(21) Anmeldenummer: 07024249.0
(22) Anmeldetag: 13.12.2007
(51) Int. Cl.: A61K 6/00, A61K 6/083

(54) **Polymerisierbares Dentalmaterial mit Initiatorsystem und Coinitiator**
Polymerizable dental material with initiator system and coinitiator
Matériau dentaire polymérisable doté d'un système initiateur et co-initiateur

(43) Veröffentlichungstag der Anmeldung: 17.06.2009
(73) Patentinhaber: Ernst Mühlbauer GmbH & Co.KG, 25870 Norderfriedrichskoog (DE)
(72) Erfinder: Lück, Rainer, Dr. sc., 25436 Tornesch (DE)
(74) Vertreter: Westphal, Thomas

(56) Entgegenhaltungen:
- EP-A- 0 923 924
- EP-A- 1 872 767
- US-A- 5 707 611

## Beschreibung

Die Erfindung betrifft ein polymerisierbares Dentalmaterial mit einem Initiatorsystem und einem Coinitiator.

Chemisch härtende polymerisierbare Dentalmaterialien (in der Literatur häufig auch selbsthärtende oder autokatalysierte Dentalmaterialien genannt) enthalten polymerisierbare Monomere, deren Polymerisation durch primär gebildete Radikale ausgelöst wird. Die Bildung dieser Radikale erfolgt durch die Reaktion eines geeigneten Initiatormoleküls, das bei Raumtemperatur allein eine ausreichende Lagerstabilität besitzt, mit einem Coinitiator. Da diese Reaktion sofort nach Zusammentreffen von Initiator und Coinitiator beginnt, müssen zur Lagerung der Dentalmaterialien beide Komponenten des Initiatorsystems getrennt untergebracht werden. Es resultieren daher, im Unterschied zu lichthärtenden Materialien, bei denen die Radikale erst durch Bestrahlung mit der blauen Komponente des sichtbaren Lichtes gebildet werden, Mehr-Komponenten-Systeme. Die Komponenten werden erst unmittelbar vor der Verarbeitung des Materials miteinander in Kontakt gebracht und innig miteinander vermischt. Diese Vermischung kann dabei entweder von Hand mit einem Mischspatel oder durch selbstanmischende Systeme (Doppelkartusche mit statischen oder dynamischen Mischkanülen) erfolgen.

Ein im Stand der Technik bei chemisch härtenden Dentalmaterialien am häufigsten verwendetes Initiatorsystem besteht aus einem zumeist aromatischen Amin und einem organischen Peroxid, wie z.B. in DE-C-97 50 72 beschrieben. Die benötigten Radikale werden bei diesem System über eine Redoxreaktion zwischen Amin und Peroxid gebildet.

Ein wesentlicher Nachteil der Amin/Peroxidsysteme besteht in der allgemein schlechten Farbstabilität. Ursache dafür sind bei Parallel-, Neben- und Folgereaktionen gebildete Produkte der Initiatorkomponenten, die aufgrund ihrer Struktur häufig gefärbt sind. Solche gefärbten Verbindung können bei der Radikalbildung entstehen, die während der Lagerung der Pasten gebildet werden oder im ausgehärteten Material zum Beispiel durch Einwirkung von sichtbarem oder UV-Licht beobachtet werden (z.B. A. Schmidt: "Kaltpolymerisate: Ein Bericht über ihre Eigenschaften, Einsatzmöglichkeiten und Vorteile", Dentallabor 11 [1970] S. 17-22). Dieser Nachteil kann bei den Amin/Peroxid-Systemen auch durch den durchaus üblichen Zusatz spezieller Licht- und UV-Stabilisatoren nicht behoben werden. Für hochästhetische Versorgungen sind diese Verfärbungen für den Patienten störend bzw. nicht akzeptabel. Daher wird für Versorgungen im Frontzahnbereich häufig auf sehr viel aufwendigere und teurere Keramik (Veneers, Kronen, Brücken, u.ä.) zurückgegriffen.

Ein weiterer Nachteil der Amin/Peroxid-Systeme besteht in der toxischen und allergieauslösenden Wirkung der Komponenten des Startersystems und deren Reaktions- und Abbauprodukten. Während des Härtungsprozesses kann eine direkte toxische Wirkung von diesen Komponenten ausgehen. Auch nach der Härtung können entsprechende nicht einpolymerisierte Moleküle durch den sauren Speichel ausgewaschen werden. Bei einer Reihe von Patienten resultieren allergische Reaktionen, die die Anwendung von Kunststoffen einschränken oder ausschließen. In Einzelfällen kann die toxische Wirkung einen anaphylaktischen (allergischen) Schock auslösen, der durchaus lebensbedrohliche Formen annehmen kann.

Weiterhin problematisch ist der Temperaturanstieg bei der Polymerisation aufgrund der exothermen Reaktionsprozesse. Amin/Peroxid gestartete Systeme polymerisieren vergleichsweise schnell und besitzen so am Gelpunkt bereits einen sehr hohen Vernetzungsgrad (Umsatz an Doppelbindungen), was eine relativ hohe Wärmemenge aus der exothermen Reaktion freisetzt. Hohe Temperaturmaxima sind die Folge. Eine zu hohe Temperatur kann aber zu Pulpaschädigungen bis hin zum Absterben des Zahnes führen.

Ein alternatives Initiatorsystem, das eine günstigere Temperaturentwicklung und eine deutlich bessere Farbstabilität besitzt, benutzt CH-acide Verbindungen in Kombination mit zweiwertigen Übergangsmetallionen und Chloridionen. Entsprechende CH-acide Verbindungen wurden intensiv von H. Bredereck und seinen Mitarbeitern untersucht (H. Bredereck et all: "Über CH-Aktive Polymerisationsinitiatoren - XIII. Mitt. Polymerisationen und Polymerisationsinitiatoren", die Makromolekulare Chemie 92 [1966] S. 70-90; H. Bredereck et all: "Polymerisationen und Polymerisationsinitiatoren - 16. Einfluß von Thio-Gruppen in Barbitursäurederivaten auf die Polymerisationsauslösung von Methacrylsäure-methylester", die Makromolekulare Chemie 176 [1975] S. 1713-1723). Von den CH-aciden Verbindungen haben sich im Dentalbereich die Barbitursäurederivate als günstig erwiesen. Sie sind in hohen Ausbeuten und Reinheiten darstellbar, industriell verfügbar (Chemische Fabrik Berg GmbH, Mainthalstr. 3, D-06749 Bitterfeld) und erlauben durch ihre Reaktionskinetik die Realisierung interessanter Eigenschaften.

Die Synthese der Barbitursäurederivate ist z.B. aus E. Fischer und A. Dilthey: "Über c-Dialkylbarbitursäuren und über die Ureide der Dialkylessigsäuren", Ann. 335 [1904] S. 335) bekannt und beschreibt die alkalische Kondensation von Derivaten des Malonsäurediethylesters mit N-substituiertem Harnstoff in Natriumalkoholat. Die dabei erhaltenen Natriumsalze der Barbitursäurederivate werden anschließend durch die Zugabe einer Säure, z.B. von Salzsäure, in die Barbitursäurederivate überführt.

Bei dem auf Barbitursäure bzw. deren Derivaten basierenden Initiatorsystem müssen die Barbitursäurederivate von den polymerisierbaren Monomeren getrennt aufbewahrt werden. Dies liegt darin begründet, dass CH-acide Verbindungen wie die Derivate der Barbitursäure bereits ohne die Mitwirkung von Cu(II)- und Chlorid-Ionen durch Autoxidation durch Luftsauerstoff Hydroperoxide bilden. Diese Hydroperoxide zerfallen unter Bildung von Radikalen, welche die Polymerisation der reaktiven Monomere initiieren, so dass es binnen kurzer Zeit zur spontanen Polymerisation kommt. Dieser spontane Polymerisationsprozess kann durch Zugabe von Stabilisatoren für kurze Zeit (im Bereich von wenigen Stunden) verzögert oder unterdrückt werden, nicht hingegen über einen längeren Zeitraum, wie es bei lagerstabilen Systemen erwünscht ist.

Stand der Technik ist hier der Ersatz reaktionsfähiger Harze in der Initiatorpaste durch solche, die unter dentalen Bedingungen durch CH-acide Verbindungen nicht zur Polymerisation gebracht werden können, oder durch Verbindungen, die keine Doppelbindungen enthalten (z.B. Polyethylenglycol).

Die erforderliche räumliche Trennung von polymerisierbaren Monomeren und CH-aciden Barbitursäurederivaten begrenzt den Anteil der polymerisierbaren Monomere im Dentalmaterial. Für fließfähige Materialien, die vorrangig aus herkömmlichen Doppelkartuschensystemen appliziert werden, kann die Zugabe nicht reaktiver Monomere durch die Verringerung der Zugabe dieser Pastenkomponente (Mischungsverhältnisse von 2:1, 4:1 und 10:1) zumindest vermindert werden. Aus diesen Gründen werden Barbitursäurederivate in Verbindung mit Cu²⁺ und Cl⁻ derzeit ausschließlich bei den fließfähigen automatisch dosierenden und anmischenden provisorischen Kronen-und Brückenmaterialien eingesetzt. Allerdings wirkt auch die geringere Menge durch die Initiatorpaste zugesetzter nicht polymerisierender Monomere als Trennmittel, was zu einer Verschlechterung der mechanischen Eigenschaften (Druckfestigkeit, Biegefestigkeit, Härte usw.) und zu einer Zunahme der Schmierschicht führt.

Hochviskose Materialien, wie z.B. modellierbare chemisch härtender Füllungskomposite sind aus Kartuschensystemen nicht ausbringbar und nicht automatisch anmischbar. Sie werden in Drehspritzen oder Dosen untergebracht und von Hand angemischt. Die Dosierung erfolgt durch den Zahnarzt ausschließlich nach Augenmaß. Dosierhilfen haben sich bisher nicht durchgesetzt. Da gleiche Materialmengen besser einschätzbar sind, als z.B. 10:1 werden diese Materialien ausschließlich im Mischungsverhältnis von 1:1 angewendet. Dieses Mischungsverhältnis ist vom Zahnarzt besser einschätzbar, als ungleiche Verhältnisse und führt so zu wesentlich kleineren Dosierfehlern und damit zu besseren Materialqualitäten. Obwohl die CH-aciden Barbitursäurederivate zu wesentlich besseren Farbstabilitäten führen, als Amin/Peroxid-Systeme, blieben diese Systeme Füllungs- und Verblendmaterialien wegen der hohen Zugabe nicht polymerisierender Monomere und der damit einhergehenden Verschlechterung der mechanischen Eigenschaften bisher verschlossen.

Überwunden wird dieses Hindernis nun mit einem Initiatorsystem, wie es das polymerisierbare Dentalmaterial gemäß Anspruch 1 umfasst. Demnach umfasst das polymerisierbare Dentalmaterial wenigstens zwei Komponenten, dass die folgenden Initiatorkomponenten umfasst:
Komponente 1 enthaltend
   a) das Salz einer CH-aciden Verbindung, wobei die CH-acide Verbindung eine radikalische Polymerisation auslösen kann, Komponente 2 enthaltend
   b) eine Säure, deren Säurestärke größer ist als die der in der in Komponente 1 als Salz vorliegenden CH-aciden Verbindung.

Kern des Initiatorsystems ist, dass im Unterschied zu den Startersystemen basierend auf CH-aciden Verbindungen aus dem Stand der Technik eine Vorstufe des aktiven Startermoleküls, nämlich ein Salz der CH-aciden Verbindung, eingesetzt wird. Die CH-acide Verbindung wird erst nach der Zugabe einer Säure, deren Säurestärke größer ist als die der als Salz vorliegenden CH-aciden Verbindung gemäß der Regel "Salz einer schwachen Säure + starke Säure ergibt Salz einer starken Säure + schwache Säure", freigesetzt und kann anschließend als Startermolekül für den Polymerisationsprozess der Monomere fungieren.

Im Unterschied zu den CH-aciden Verbindungen, wie sie in Startersystemen des Standes der Technik verwendet wurden, ist das Salz der CH-aciden Verbindung auch über längere Zeiträume lagerstabil. Damit wird die Initiatoraktivität für die Polymerreaktion der polymerisierbaren Monomere auch bei längerer Lagerung der Komponenten des polymerisierbaren Dentalmaterials sichergestellt. So können die Pasten, aus denen das polymerisierbare Dentalmaterial angemischt wird, und insbesondere die das Salz der CH-aciden Verbindung enthaltende Paste, mehr als 3 Monate, vorzugsweise mehr als 6 Monate, besonders bevorzugt mehr als 24 Monate farb-und/oder lagerstabil aufbewahrt werden.

Ausgehend von dem Startersystem mit den Initiatorkomponenten gemäß Anspruch 1, können in eine oder beide Komponenten des Dentalmaterials polymerisierbare Monomere eingebracht werden. Damit kann die eingangs erläuterte unerwünschte Beschränkung der Menge an polymerisierbaren Monomeren im polymerisierbaren Dentalmaterial, d. h. der Anteil der Polymermatrix im Dentalmaterial, aufgehoben werden. Daraus ergeben sich auch vorteilhafte mechanische Eigenschaften des polymerisierten Dentalmaterials, da die Menge der nicht reaktiven Monomere (z.B. Monomere mit nicht reaktiven Doppelbindungen oder Verbindungen, die keine Doppelbindungen enthalten), die üblicherweise aus Gründen der Handhabbarkeit der Initiatorpaste zugefügt werden (z. B. zur Einstellung pastöser Eigenschaften um die Paste in Kartuschensystemen verwenden zu können), verringert bzw. ganz darauf verzichtet werden kann. Es ist bekannt, das nicht einpolymerisierende Harze oder Füllstoffe in den Basis- und Initiatorpasten wie Trennmittel wirken und je nach Gehalt die mechanischen Eigenschaften nachteilig beeinflussen. Dieser Effekt wird bei der Verwendung des erfindungsgemäßen Startersystems weitestgehend vermieden, bei dem gemäß einer bevorzugten Ausführungsform der Erfindung die Monomere beider Komponenten bei deren Mischung radikalisch polymerisieren.

Werden die beiden Initiatorkomponenten gemäß Anspruch 1 miteinander in Verbindung gebracht, polymerisieren die polymerisierbaren Monomere und das Material härtet aus. Die Polymerisationszeit wird durch die Erhärtungszeit (EHZ), definiert durch den Erhärtungsanfang (EA) und das Erhärtungsende (EE), charakterisiert. Um das Verhältnis von EA zu EE, d.h. den Übergang vom fließfähigen in den abgebundenen Zustand zu beschreiben, ist auch der Begriff des "Snap-Set" gebräuchlich.

Die Erfindung hat erkannt, dass die durch das Startersystem gemäß Anspruch 1 vorgegebene Polymerisationszeit für die Verwendung in radikalisch polymerisierbarem Dentalmaterial in bestimmten Situationen vorteilhafterweise mit einem Coinitiator gemäß Anspruch 1 gesteuert, insbesondere verkürzt werden kann.

Die Erfindung hat des weiteren erkannt, dass sich mit den erfindungsgemäßen Alkyl(aryl)ammoniumhalogeniden als Coinitiator zu den Initiatorkomponenten gemäß Anspruch 1 eine kurze Polymerisationszeit (Snap-Set) einstellen lässt, ohne dass darunter die für Dentalmaterialien erforderlichen mechanischen Eigenschaften, charakterisiert beispielsweise durch die Biegefestigkeit und das Biegemodul, leiden. Dies wird im Einzelnen durch die in den Beispielen 8-15 ausgeführten Untersuchungen belegt und lässt sich nachfolgend wie folgt zusammenfassen:
Nicht alle Alkyl(aryl)ammoniumhalogenide sind für die Verwendung als Coinitiator geeignet.
Mit zunehmender Länge der Alkylgruppen verschlechtert sich das Snap-Set, wobei die Grenze bei mehr als einer C12-Alkylgruppe als Ligand liegt.
Bei Einführung einer Benzylgruppe verbessert sich mit der N-Alkyl-Kettenlänge der übrigen Liganden das Snap-Set.
Verbindungen mit mehr als einer C12-Alkylgruppen sind sowohl in Hinblick auf die Erhärtungszeiten, als auch die mechanischen Eigenschaften des Dentalmaterials (Biegemodul und Biegefestigkeit) ungeeignet.
Das Anion des erfindungsgemäßen Coinitiators beeinflusst ebenfalls die Erhärtungszeit und die mechanischen Eigenschaften des Dentalmaterials. So sind Chloride am besten, Iodide weniger, Bromide noch weniger geeignet und Fluoride(Dihydrate)ungeeignet.

Basierend auf den vorgenannten Aussagen, die durch die entsprechenden Untersuchungen gemäß den Beispielen 8 - 15 belegt sind, wird ein Coinitiator für radikalisch polymerisierende Dentalmaterialien gemäß Anspruch 1 der Formel 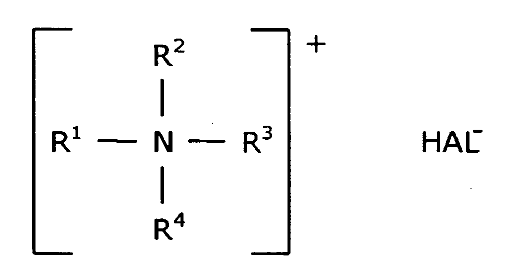 beansprucht mit
HAL⁻ = Cl⁻, I⁻ oder Br⁻ und
R¹, R², R³ und R⁴ = lineares oder verzweigtes Alkyl mit
1 bis 12 C-Atomen, vorzugsweise 2 bis 6 C-Atomen, oder
R¹, R² und R³ = lineares oder verzweigtes Alkyl mit 1 bis 12 C-Atomen, vorzugsweise 2 bis 6 C-Atomen und R⁴ = Benzyl,
wobei höchstens eine R-Gruppe eine C₁₂-Alkylgruppe ist, wobei die Coinitiatorverbindung nicht Lauryltrimethylammoniumchlorid oder Lauryldimethylbenzylammoniumchlorid ist. Lauryltrimethylammoniumchlorid und Lauryldimethylbenzylammoniumchlorid sind in EP-A-0 923 924 als fakultative Tensidkomponente eines Klebstoffsets beschrieben.

Nachfolgend werden bevorzugte Ausführungsformen der Erfindung beschrieben.

Die Coinitiatorverbindung ist gemäß einer bevorzugten Ausführungsform der Erfindung ausgewählt aus der Gruppe bestehend aus Benzyl-trimethyl-ammoniumchlorid und Benzyl-tributyl-ammoniumchlorid.

Bezogen auf das Dentalmaterial beträgt die Menge der Coinitiatorverbindung, beispielsweise Benzyl-trimethyl-ammoniumchlorid oder Benzyl-tributyl-ammoniumchlorid, 0,3 - 5 Gew.-%, vorzugsweise zwischen 0,3 und 2,5 Gew.-%, besonders bevorzugt zwischen 0,3 und 0,65 Gew.-%.

Bezogen auf eine der Komponenten 1 oder 2, die beispielsweise im Verhältnis 1:1 angemischt werden können, beträgt die Menge der Coinitiatorverbindung, beispielsweise Benzyl-trimethyl-ammoniumchlorid oder Benzyl-tributyl-ammoniumchlorid, 0,6 - 10 Gew.-%, vorzugsweise zwischen 0,6 und 5 Gew.-%, besonders bevorzugt zwischen 0,6 und 1,3 Gew.-%.

Die Coinitiatorverbindung liegt in der Komponente 1 und/oder der Komponente 2, vorzugsweise in der Komponente 1, vor.

Erfindungsgemäß bevorzugte radikalisch polymerisierbare Monomere sind aus der Gruppe der Acrylsäureester und Methacrylsäureester ausgewählt.

Das erfindungsgemäße polymerisierbare Dentalmaterial enthält vorzugsweise insgesamt über 50 Gew.-%, vorzugsweise über 60 Gew.-%, weiter vorzugsweise über 70 Gew.-%, weiter vorzugsweise über 80 Gew.-%, weiter vorzugsweise über 90 Gew.-%, weiter vorzugsweise über 95 Gew.-%, weiter vorzugsweise über 98 Gew.-% radikalisch polymerisierbare Monomere. Gemäß einer bevorzugten Ausführungsform der Erfindung enthält die Komponente 1 des polymerisierbaren Dentalmaterials über 50 Gew.-%, vorzugsweise über 60 Gew.-%, weiter vorzugsweise über 70 Gew.-%, weiter vorzugsweise über 80 Gew.-%, weiter vorzugsweise über 90 Gew.-%, weiter vorzugsweise über 95 Gew.-%, weiter vorzugsweise über 98 Gew.-% radikalisch polymerisierbare Monomere. Gemäß einer bevorzugten Ausführungsform der Erfindung enthält die Komponente 1 und/oder die Komponente 2 oder das polymerisierbare Dentalmaterial insgesamt keine nicht radikalisch polymerisierbaren Monomere.

Das erfindungsgemäße polymerisierbare Dentalmaterial kann die Komponente 1 in einer ersten Paste und die Komponente 2 in einer zweiten Paste enthalten und in einem vorgesehenen Mischungsverhältnis beider Pasten von 1:10 oder größer, vorzugsweise 1:4 oder größer, weiter vorzugsweise 1:2 oder größer, besonders bevorzugt 1:1 angemischt werden.

Als Salz der CH-aciden Verbindung der Komponente 1 sind insbesondere Salze der α-Benzoyl-proprionitrile, α -Cyancarbonsäureester und -amide, cyclischer β-Oxonitrile, β -Diketone, cyclischer β-Diketone, cyclischer β -Oxocarbonsäureester, cyclischer β-Oxo-lactone, Malonsäure, Malonsäurederivate, Pyrazolderivate, Barbitursäure oder Barbitursäurederivate geeignet.

Das Salz der CH-aciden Verbindung ist vorzugsweise ein Salz ausgewählt aus der Gruppe bestehend aus einwertigen und zweiwertigen Salzen der Alkali- und Erdalkaliionen. Das Salz der CH-aciden Verbindung kann beispielsweise ein Natriumsalz sein.

Als Säure der Komponente 2 im Sinne vorliegender Erfindung kann eine organische oder anorganische Säure verwendet werden, sofern deren Säurestärke größer ist als die der in der Komponente 1 als Salz vorliegenden CH-aciden Verbindung.

Als anorganische Säuren kommen nicht oxydierende Säuren wie z.B. Salzsäure oder Phosphorsäure in Betracht.

Besonders geeignete organische Säuren sind Monocarbonsäuren, ausgewählt aus der Gruppe bestehend aus Ameisensäure, Essigsäure und Benzoesäure bzw. Derivate dieser Säuren oder Dicarbonsäuren, ausgewählt aus der Gruppe bestehend aus Oxalsäure, Malonsäure, Succinsäure, Adipinsäure, Pimelinsäure, Azelainsäure, Sebacinsäure, Maleinsäure, Fumarsäure, Sorbinsäure, Phthalsäure und Terephthalsäure bzw. Derivate dieser Säuren oder Tricarbonsäuren, ausgewählt aus der Gruppe bestehend aus Hemimellithsäure, Trimellithsäure, Trimesinsäure, Agaricinsäure, Citronensäure, 1,2,3-Propantricarbonsäure bzw. Derivate dieser Säuren oder Multicarbonsäuren, ausgewählt aus der Gruppe bestehend aus Pyromellithsäure und Mellithsäure bzw. Derivate dieser Säuren oder Polycarbonsäuren, ausgewählt aus der Gruppe bestehend aus Polyacrylsäure und Polymethacrylsäure bzw. Derivate dieser Säuren. Die Säuren können eine oder mehrere polymerisierbare Gruppen, beispielsweise (Meth)acrylgruppen enthalten.

Der sich auf das Dissoziationsgleichgewicht in einem wässrigen Medium beziehende pKₐ-Wert kann dabei als ein Maß für die CH-Acidität zumindest mit herangezogen werden. Eine Auswahl an organischen Säuren im Sinne vorliegender Erfindung, deren pKₐ-Wert kleiner als der pKₐ-Wert der Barbitursäure (4,01) ist, sind beispielsweise 2,5-Dihydroxybenzoesäure (2,97), Fumarsäure (3,03), Maleinsäure (1,83), Phtalsäure (2,89), Salicylsäure (2,97), 2,4,6-Trihydroxybenzoesäure (1,68) und Zimtsäure (3,89).

Das erfindungsgemäße polymerisierbare Dentalmaterial kann in mindestens einer der Komponenten des polymerisierbaren Dentalmaterials Übergangsmetallkationen, vorzugsweise Cu²⁺-Ionen, und zur Radikalbildung geeignete Anionen, vorzugsweise Halogenid-Ionen, weiter vorzugsweise Chlorid-Ionen, enthalten, die den Polymerisationsprozess initiieren, steuern und beschleunigen. Diese Übergangsmetallkationen, vorzugsweise Cu²⁺-Ionen, und zur Radikalbildung geeigneten Anionen, vorzugsweise Halogenid-Ionen, weiter vorzugsweise Chlorid-Ionen, liegen vorzugsweise in der Basispaste (Komponente 1) des anspruchsgemäßen polymerisierbaren Dentalmaterials vor, können aber je nach Erfordernis auch der Inititiatorpaste (Komponente 2) zugesetzt werden. Weiter bevorzugte Kupferverbindungen sind Kupferacetat,
- acetylacetonat, -naphthenat, -salicylat, -oleat, -stearat,
- citrat oder -(meth)acrylat.

Das erfindungsgemäße Dentalmaterial kann in mindestens einer der Komponenten Füllstoffe enthalten. Bei den erfindungsgemäß eingesetzten Füllstoffen handelt es sich bevorzugt um nano- und/oder mikroskalige (teilweise röntgenopake) Füllstoffe, vorzugsweise um Glaspulver, Glaskeramikpulver, Metall-, Halbmetall- oder Mischmetalloxide, Silikat-, Nitrid-, Sulfat-, Titanat-, Zirkonat-, Stannat-, Woframat-, Siliciumdioxid- Verbindungen oder eine Mischung aus diesen Verbindungen oder sphärische Füllstoffe, Quarzpulver oder eine Mischung aus diesen Pulvern oder gefüllte oder ungefüllten Splitterpolymerisate und/oder Perlpolymerisate.
Bei den erfindungsgemäß eingesetzten nanoskaligen Füllstoffen handelt es sich besonders bevorzugt um Siliziumdioxid, Aluminiumoxid, Zirkondioxid, Titandioxid, Zinkoxid, Zinndioxid, Ceroxid, Aluminium-Silizium-Oxide, Silizium-Zink-Oxide, Silizium-Zirkon-Oxide, Eisenoxide und deren Mischungen mit Siliziumdioxid, Indiumoxide und deren Mischungen mit Siliziumdioxid und/oder Zinndioxid, Bornitrid, Strontiumsulfat, Bariumsulfat, Strontiumtitanat, Bariumtitanat, Natriumzirkonat, Kaliumzirkonat, Magnesiumzirkonat, Calciumzirkonat, Strontiumzirkonat, Bariumzirkonat, Natriumwolframat, Kaliumwolframat, Magnesiumwolframat, Calciumwolframat, Strontiumwolframat und/oder Bariumwolframat.

Der Füllstoff kann gemäß einer bevorzugten Ausführungsform der Erfindung ein oberflächenmodifizierter Füllstoff, vorzugsweise eine organisch oberflächenmodifizierter Füllstoff sein. Der Füllstoff kann nach seiner Oberflächenmodifikation, beispielsweise einer Silanisierung, auf seiner Oberfläche funktionelle Gruppen, beispielsweise reaktive Methacrylatgruppen besitzen, die mit den Monomeren chemisch, vorzugsweise radikalisch, reagieren können oder eine hohe Affinität zu der aus den Monomeren gebildeten Polymermatrix aufweisen.

Das erfindungsgemäße Dentalmaterial kann zur Einstellung bestimmter Eigenschaften zusätzlich so genannte Additive bzw. Modifikatoren enthalten. Die Allgemeinheit nicht einschränkend sind nachfolgend einige Beispiele genannt:
anorganische und/oder organische Farbpigmente bzw. Farbstoffe, Stabilisatoren (wie z. B. substituierte und unsubstituierte Hydroxyaromaten, Tinuvine, Terpinene, Phenothiazin, sogenannte HALS - Hindered Amine Light Stabilizers - und/oder Schwermetallfänger wie EDTA), Weichmacher (wie z. B. Polethylenglykole, Polypropylenglykole, ungesättigte Polyester, Phthalate, Adipate, Sebacate, Phosphorsäureester, Phosphonsäureester und/oder Zitronensäureester), ionenabgebende Substanzen, insbesondere solche die Fluoridionen freisetzen (wie z. B. Natriumfluorid, Kaliumfluorid, Yttriumfluorid, Ytterbiumfluorid und/oder quartäre Ammoniumfluoride), bakterizide oder antibiotisch wirksame Substanzen (wie z. B. Chlorhexidin, Pyridiniumsalze, Penicilline, Tetracycline, Chloramphenicol, antibakterielle Makrolide und/oder Polypeptid-Antibiotika) und/oder Lösungsmittel (wie z. B. Wasser, Aceton, Ethanol, i-Propanol, Butanon und/oder Essigsäureethylester).
Das erfindungsgemäße Dentalmaterial kann für die prothetische, konservierende und präventive Zahnheilkunde verwendet wenden. Ohne Anspruch auf Vollständigkeit seien stellvertretend einige Anwendungsbeispiele genannt:
   Füllungsmaterial, Stumpfaufbaumaterial, Befestigungsmaterial, provisorisches und permanentes Kronen- und Brückenmaterial, Bonding-Materialien, zahntechnischer Werkstoff zur Herstellung von Inlays, Onlays, Verblendschalen, künstlichen Zähnen, Modellmaterialien und Fissuren- und Wurzelkanal-Versiegelungsmaterial.

Die Erfindung wird nachfolgend ohne Einschränkung der Allgemeinheit anhand von Ausführungsbeispielen erläutert, wobei sich die Beispiele 1 bis 7 in Sektion I mit dem Initiatorsystem und die Beispiele 8 bis 15 in der Sektion II mit dem erfindungsgemäßen Coinitiator zu dem Initiatorsystem beschäftigen.

### Sektion I

### Beispiel 1: Synthese des Natriumsalzes der 1,3,5-Trimethylbarbitursäure

Die Synthese wurde von A.C.Cope et al.: "1,3-Dimethyl-5-alkyl Barbituric Acids": J.Amer.Chen.Soc. 63 365 (1941)beschrieben. 0,1 mol (=17,420g) Methylmalonsäurediethyl-ester wurden zu 97,214 g 21%-ige Natriumalkoholatlösung in Ethanol (= 0,3 mol Natriumalkoholat) gegeben und beide Komponenten innig miteinander vermischt. Dabei entstehen Natrium-methylmalonsäure-diethylester und Ethanol. Anschließend wurde 0,1 mol (= 8,811 g) N, N-Dimethylharnstoff in 15 ml Ethanol (p.a.) gelöst und zur Lösung langsam zugetropft, wobei das Natriumsalz der 1,3,5-Trimethylbarbitursäure entsteht. Anschließend wurde der Ansatz 11,5 Stunden unter Rückfluß gekocht.

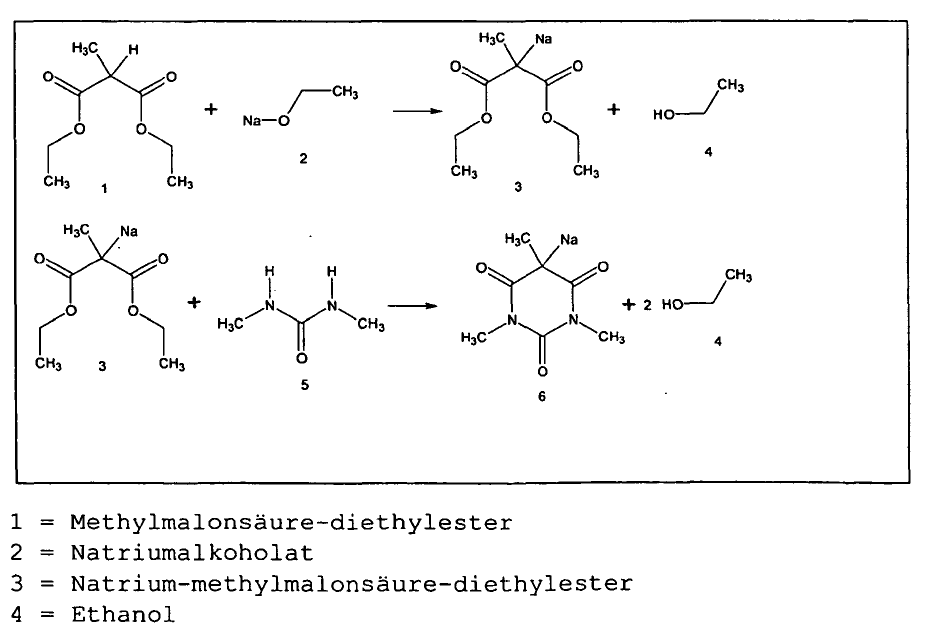

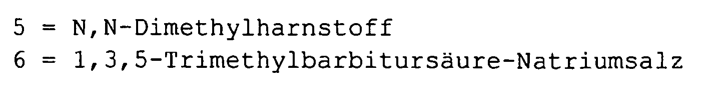

Die Lösung wurde am Rotationsverdampfer bis zur Trockne eingeengt und der Rückstand mit 100 ml deionisiertem Wasser aufgenommen. Danach wurde 5 Mal mit je 20 ml Ether ausgeschüttelt. Die wässrige Phase wurde am Rotationsverdampfer erneut bis zur Trockne eingeengt und anschließend in eine Filter-nutsche überführt. Hier wurde der Rückstand mit Isopropanol gewaschen, bis das abgesaugte Isopropanol keine Färbung mehr aufwies. Da der Feststoff immer noch leicht bräunlich gefärbt war, wurde mit wenig Ethanol (p.a.) gewaschen, bis auch das Ethanol keine Färbung mehr aufwies.
Die Reinheit wurde mittels HPLC mit 99,59% bestimmt. Die Ausbeute betrug 65,30%.

### Beispiel 2: Herstellung und Lagerstabilität der Initiatorpaste

Es wurden drei unterschiedliche Initiatorpasten hergestellt und auf ihre Lagerstabilität bei Raumtemperatur und bei 40°C untersucht:

| | |
|---|---|
| Paste 1: | Paste mit Polyethylenglycol mit mittlerem Molekulargewicht von 400 g/mol (PEG 400) als unreaktive Harzkomponente und mit Dentalglas gefüllt, das mit methacrylatgruppenfreiem Silan oberflächenbehandelt war und 1,3,5-Trimethylbarbitursäure als Startermolekül enthielt. |
| Paste 2: | Paste mit Methacrylaten und mit Dentalglas gefüllt, das mit Methacrylatgruppen tragendem Silan oberflächenbehandelt war und 1,3,5-Trimethylbarbitursäure als Startermolekül enthielt. |
| Paste 3: | Paste mit Methacrylaten und mit Dentalglas gefüllt, das mit Methacrylatgruppen tragendem Silan oberflächenbehandelt war und das Natriumsalz der 1,3,5-Trimethylbarbitursäure als Startermolekül enthielt. |

Die folgende Tabelle zeigt die Rezepturen der Pasten. Dabei war die zugegebene Startermolekülmenge so berechnet, dass alle drei Pasten gleiche Molzahlen enthielten.

| Bestandteil | Paste 1 | Paste 2 | Paste 3 |
|---|---|---|---|
| | Gew.-% | Gew.-% | Gew.-% |
| Bis-GMA | - | 37,2179 | 36,9790 |
| TEDMA | - | 16,7211 | 16,6138 |
| PEG 400 | 53,9390 | - | - |
| Aerosil R812 | 1,5000 | 1,5000 | 1,4904 |
| Dentalglas sil. ohne Methacrylatgruppen | 39,9350 | - | - |
| Dentalglas sil. mit Methacrylatgruppen | - | 39,9350 | 39,6785 |
| 1,3,5-Trimethylbarbitursäure | 4,6260 | 4,6260 | - |
| 1,3,5-Trimethylbarbitursäure Natriumsalz | - | - | 5,2383 |

Um eine Aussage über die Lagerstabilität der Pasten zu erhalten, wurden die Pasten bei Raumtemperatur und bei 40°C eingelagert und in regelmäßigen Abständen auf polymerisierte Anteile untersucht. Während die Paste 2 bereits nach 90 min vollständig auspolymerisiert war, zeigen die Pasten 1 und 3 auch nach mehr als 6 Monaten noch keinerlei Anzeichen von Polymerisation. Das zeigt, dass Pasten mit reaktiven Monomeren und 1,3,5-Trimethylbarbitursäure als aktives Startermolekül nicht lagerstabil sind, während bei Einsatz des Natriumsalzes der 1,3,5-Trimethylbarbitursäure als Vorstufe des aktiven Startermoleküls 1,3,5-Trimethylbarbitursäure lagerstabile Pasten resultieren.

### Beispiel 3: Untersuchung der Reaktionsfähigkeit der Initiatorpasten aus Beispiel 2

Um die Reaktionsfähigkeit der im Beispiel 2 hergestellten Initiatorpasten zu untersuchen, wurden diese von Hand mit der Basispaste des Produktes Luxatemp Automix A2 der Fa. DMG Hamburg im Verhältnis 10 : 1 (10 Teile Basispaste, 1 Teil Initiatorpaste) angemischt und die Erhärtungszeit bestimmt.

Mit der Polyethylenglykolhaltigen Paste 1 begann die Erhärtung nach 1:40 min. und war nach 3:20 min. abgeschlossen. Das bedeutet, dass diese Paste lagerstabil und reaktionsfähig ist.

Bei Verwendung der Initiatorpaste 2 ergibt sich ein Erhärtungsbeginn von 1:50 min. die Erhärtung ist hier ebenfalls nach ca. 3:20 min. abgeschlossen. D.h. die Paste besitzt eine ausreichende Reaktivität, ist aber, wie Beispiel 2 zeigte, nicht lagerstabil. Sie ist daher als Initiatorpaste nicht einsetzbar.

Die Initiatorpaste 3, die reaktive Methacrylgruppen und das Natriumsalz der 1,3,5-Trimethylbarbitursäure enthält, erhärtete nach dem Anmischen mit der oben genannten Basis erwartungsgemäß nicht. Deshalb wurde dem angemischten System (0,6g Basispaste + 0,06g Initiatorpaste) 1 Tropfen 32%-ige Salzsäure zugesetzt. Nach 55 min. polymerisierte das Material.

### Beispiel 4: Reaktion der Initiatorpaste 3 aus Beispiel 2 mit Salzsäure bzw. einer Salzsäure enthaltenden Basispaste

Die Initiatorpaste 3 aus Beispiel 2, die das Natriumsalz der 1,3,5-Trimethylbarbitursäure und reaktive Methacrylatmonomere enthält, wurde mit einem Tropfen Salzsäure gemischt. Nach ca. 90 Minuten war die Paste ausgehärtet.

In einem zweiten Versuch wurde der Initiatorpaste 3 aus Beispiel 2 neben dem Tropfen Salzsäure die reaktive Methacrylatmonomere und Coinitiatoren enthaltende Basispaste des Produktes Luxatemp Automix der Fa. DMG Hamburg im Verhältnis 1:1 zur Initiatorpaste zugemischt. Die erhaltene Paste polymerisierte nach 20 Minuten.

Die Versuche belegen, dass das Natriumsalz des Barbitursäurederivats *in situ* in die freie Säure nach Zugabe der Salzsäure überführt wird und nach Aufbau der CH-Acidität die Pasten infolge der sich anschließenden Autoxidation nach kurzer Zeit polymerisieren. Die Versuche zeigen des Weiteren, dass durch in der Basispaste enthaltene Coinitiatoren, beispielsweise Alkylammoniumchloride und Cu(II)-Verbindungen, wie sie in der Basispaste des Produktes Luxatemp Automix enthalten sind, die Polymerisationszeiten erheblich verkürzen können.

### Beispiel 5: Reaktion der Initiatorpaste 3 aus Beispiel 2 mit organischen Säuren

Es wurden eine Auswahl an organischen Säuren, deren pKₐ-Wert unter dem der Barbitursäure liegt, auf Ihre Fähigkeit geprüft, das Natriumsalz der Paste 3 gemäß Beispiel 2 in die freie 1,3,5-Trimethylbarbitursäure zu überführen. Als Maß für die Überführbarkeit diente die Erhärtungszeit des Pastengemisches. Die Konzentration der verwendeten organischen Säuren wurde so berechnet, dass sie äquimolar zu der in der Initiatorpaste eingesetzten Molzahl des Startermoleküls bei einer Anmischung von 10:1 war. Bei der Anmischung der Initiatorpaste mit den nachfolgend genannten organischen Säuren 2,5-Dihydroxybenzoesäure, Fumarsäure, Maleinsäure, Phtalsäure, Salicylsäure, 2,4,6-Trihydroxybenzoesäure und Zimtsäure

war innerhalb von 30 Minuten eine Vergelung, d.h. Polymerisation, zu beobachten.

Beispielhaft sei dies nachfolgend für die Reaktivität der Initiatorpaste 3 aus Beispiel 2 nach Zugabe von Fumarsäure bzw. 2,5-Dihydroxybenzoesäure beschrieben.

0,5g der oben genannten Basispaste wurden mit 0,05g der Initiatorpaste 3 aus Beispiel 2 vermischt und 1 Tropfen in Hydroxyethylmethacrylat gelöste Fumarsäure (0,1g in 10 ml) zugegeben. Das Material polymerisierte nach 6 Minuten.

In einem weiteren Versuch wurde nun die Fumarsäurelösung in die Basispaste eingearbeitet. Dabei wurde ein Molverhältnis von Fumarsäure: 1,3,5-Trihydroxybarbitursäurenatriumsalz von 1:1 (nach Anmischung der Pasten im Verhältnis von 10:1) eingestellt. Das Material polymerisierte nach 19 Minuten.

In einem weiteren Versuch wurde die in Hydroxyethylmethacrylat besser lösliche 2,5-Dihydroxybenzoesäure eingesetzt. Dazu wurde 0,1g der 2,5-Dihydroxybenzoesäure in 1 ml Hydroxyethylmethacrylat gelöst. 1 Tropfen der Lösung (0,0248g) wurde in 0,5g der oben genannten Basispaste eingearbeitet. Anschließend wurden 0,05g der Initiatorpaste 3 aus Beispiel 2 zugegeben und gründlich durchmischt. Nach 9 min. war das Pastengemisch erhärtet.

### Beispiel 6 (erfindungsgemäß)

Um die Reaktionsfähigkeit und die Biegefestigkeit als Maß für die mechanische Festigkeit des erfindungsgemäßen Dentalmaterials zu untersuchen, wurde die nachfolgend spezifizierte Initiatorpaste 4 hergestellt und mit der Basispaste aus Beispiel 3, in die 1,53 Gew.-% 2,5-Dihydroxybenzoesäure eingearbeitet wurde, im Verhältnis 1:1 angemischt.

| Bestandteil | Paste 4 |
|---|---|
| | Gew.-% |
| Bis-GMA | 38,5 |
| TEDMA | 17,1 |
| PEG 400 | - |
| Aerosil R812(oberflächenbehan delte Pyrogene Kieselsäure) | 1,6 |
| Dentalglas sil. mit Methacry latgruppen (D50: 1,5 µm) | 42,1 |
| 1,3,5-Trimethylbarbitursäure | - |
| Na-1,3,5-Trimethylbarbiturat | 0,7 |

Die Erhärtung begann nach 2:30 min und war nach 5:30 min. abgeschlossen. Die mittlere Biegefestigkeit betrug 72,11 MPa (+/- 6,00, 10 Messungen). Die maximale Biegefestigkeit betrug 81,97 MPa.

### Beispiel 7 (Vergleichsbeispiel)

Um die Biegefestigkeit eines nicht erfindungsgemäßen Dentalmaterials zu untersuchen, wurde die nachfolgend spezifizierte Initiatorpaste 5 hergestellt und mit der Basispaste aus Beispiel 3 im Verhältnis 1:1 angemischt.

| Bestandteil | Paste 5 |
|---|---|
| | Gew.-% |
| Bis-GMA | - |
| TEDMA | - |
| PEG 400 | 56,1 |
| Aerosil R812(oberflächenbehandelte Pyrogene Kieselsäure) | 1,6 |
| Dentalglas sil. ohne Methacrylatgruppen (D50: 1,5 µm) | 41,5 |
| 1,3,5-Trimethylbarbitursäure | 0,8 |
| Na-1,3,5-Trimethylbarbiturat | - |

Die mittlere Biegefestigkeit des ausgehärteten Dentalmaterials betrug 16,89 MPa (+/- 2,25, 10 Messungen) und die maximale Biegefestigkeit betrug 19,03 MPa.

Die Biegefestigkeit des Dentalmaterials des erfindungsgemäßen Beispiels 6 mit einer mittleren Biegefestigkeit von 72,11 MPa und einer maximale Biegefestigkeit 81,97 MPa lag damit deutlich über den entsprechenden Werten des Vergleichsbeispiels 7 und belegt die extreme Belastbarkeit des erfindungsgemäßen Dentalmaterials.

### Sektion II

Mit den nachfolgenden Beispielen wird gezeigt, dass die Struktur des Tetraalkyl(aryl)ammoniumkations und das Halogenion des Coinitiators, als auch seine Konzentration einen erheblichen Einfluss auf die Erhärtungscharakteristik und mechanischen Eigenschaften des erfindungsgemäßen Dentalmaterials aufweisen.

### Beispiel 8

Zur Durchführung der nachfolgenden Untersuchungen wurde ein Pastensystem gewählt, bei dem eine Basis und Initiatorpaste im Verhältnis 1:1 angemischt werden. Die folgende Tabelle zeigt die Rezepturen beider Pasten.

| Bestandteil | Basispaste | Initiatorpaste |
|---|---|---|
| | [Gew.-%] | [Gew.-%] |
| Bis-GMA | 33,2716 | 38,3791 |
| TEGDMA | 23,9113 | 15,2190 |
| Dentalglas sil. mit Methacrylatgruppen | 32,5918 | 41,1819 |
| Aerosil | 3,8020 | 1,6045 |
| Tetraalkyl(aryl)ammoniumchlorid (Benzyl-tributylammoniumchlorid) | (4,8976) | - |
| 2,5-Dihydroxybenzoesäure | 1,5257 | - |
| Cu-Acetylacetonat | - | 0,0053 |
| Na-1,3,5-Trimethylbarbiturat | - | 1,3307 |

Bei Einsatz von Benzyl-tributyl-ammoniumchlorid (15,7226 mmol = 4,8976 Gew.-% der Basispaste)als Tetraal-kyl(aryl)ammoniumchlorid ergibt sich eine Erhärtungszeit von 147 / 324 s (EA/EE) bzw. 151 / 345 s und eine Biegefestigkeit von 69 ± 6 MPa (Maximalwert: 77 MPa) und ein Biegemodul von 2001 ± 118 MPa (Maximalwert: 2174 MPa).

Bei den nachfolgenden Untersuchungen weiterer Tetraal-kyl(aryl)ammoniumsalze wurde wie folgt vorgegangen:
1. Es wurden bei allen Versuchen 15,7226 mmol Tetraal-kyl(aryl)ammoniumsalz eingesetzt. Alle anderen Komponenten des Initiatorsystems wurden konstant gehalten.
2. Harze, Gläser und Aerosil wurden zu einer 100%-Rezeptur hochgerechnet.
3. Die Initiatorpaste blieb für alle Versuche konstant.

### Beispiel 9

Zunächst wurde der Einfluss der Kettenlänge von 4 gleichen Alkylketten am Stickstoff des Tetraalkylammoniumchlorids auf die Aushärtungscharakteristik und die mechanischen Eigenschaften (Biegemodul und Biegefestigkeit bestimmt nach DIN EN ISO 4049) untersucht. Die Kettenlänge wurde zwischen Ethyl (C₂) und Hexyl (C₆)variiert. Die Ergebnisse der Untersuchung sind in der nachfolgenden Tabelle zusammengefasst.

| Tetraalkylammoniumchlorid Molmasse [g/mol] | EHZ [s] EA / EE | Biegefestigkeit [MPa] Maximalwert Mittelwert | Biegemodul [MPa] Maximalwert Mittelwert |
|---|---|---|---|
| Tetra**ethyl** | 63 / 178 | Xₘₐₓ = 77 | Xₘₐₓ = 2377 |
| 166 | 63 / 174 | ∅ = 71 ± 6 | ∅ = 2135 ± 182 |
| Tetra**butyl** | 105 / 228 | Xₘₐₓ = 78 | Xₘₐₓ = 2809 |
| 278 | 114 / 226 | ∅ = 71 ± 5 | ∅ = 2306 ± 201 |
| Tetra**pentyl** | 86 / 231 | Xₘₐₓ = 84 | Xₘₐₓ = 2749 |
| 334 | 91 / 234 | ∅ = 78 ± 6 | ∅ = 2460 ± 240 |
| Tetra**hexyl** | 99 / 231 | Xₘₐₓ = 79 | Xₘₐₓ = 2400 |
| 390 | 108 / 213 | ∅ = 76 ± 3 | ∅ = 2228 ± 149 |

Wie sich aus den Tabellenwerten ergibt, steigen die Erhärtungszeiten bei den längerkettigen Liganden (Butyl, Pentyl und Hexyl) gegenüber den kürzerkettigen Liganden (Ethyl) an. Bei den längerkettigen Liganden sind Erhärtungsanfang und -ende und Snap-Set im Wesentlichen gleich. Die Biegefestigkeit und das Biegemodul sind ähnlich, d.h. von der Länge der Alkylketten unabhängig.

### Beispiel 10

In einem weiteren Versuch wurde der Einfluss eines aromatischen Liganden im Tetraalkyl(aryl)-Ion untersucht.

Dafür wurde eine der Alkylketten im Molekül durch einen Benzylrest ersetzt und die entsprechenden Benzyltrialkylammoniumsalze gemäß nachfolgender Tabelle auf EHZ, Biegefestigkeit und Biegemodul untersucht.

| Alkyl(aryl) ammoniumchlorid Molmasse [g/mol] | EHZ [s] EA / EE | Biege- festigkeit [MPa] Maximalwert Mittelwert | Biegemodul [MPa] Maximalwert Mittelwert |
|---|---|---|---|
| Benzyl-tri-**methyl** | 63 / 216 | Xₘₐₓ = 47 | Xₘₐₓ = 1321 |
| 185 | 62 / 207 | ∅ = 44 ± 3 | ∅ = 1200 ± 100 |
| Benzyl-tri-**ethyl** | 118 / 336 | Xₘₐₓ = 58 | Xₘₐₓ = 1452 |
| 227 | | ∅ = 52 ± 4 | ∅ = 1280 ± 95 |
| Benzyl-tri-**butyl** | 147 / 324 | Xₘₐₓ = 78 | Xₘₐₓ = 2174 |
| 311 | 151 / 345 | ∅ = 69 ± 6 | ∅ = 2002 ± 118 |

Wie sich aus den Tabellenwerten ergibt, steigen die Erhärtungszeiten mit zunehmender Kettenlänge an, wobei Erhärtungsbeginn als auch Erhärtungsende einen exponentiellen Verlauf zeigen. Mit Zunahme der Alkylkettenlänge sind höhere Biegefestigkeit- und Biegemodulwerte zu erkennen.

### Beispiel 11

Die sich aus den vorgenannten Beispielen ergebende Tendenz, dass längere Ketten zu längeren Erhärtungszeiten führen und mit einer höherer Biegefestigkeit und höherem Biegemodul einhergehen können, bestätigen ebenfalls die in der nachfolgenden Tabelle zusammengefassten Ergebnisse des Vergleiches von Benzyl-tri-methylammoniumchlorid mit Benzyl-dimethyl-dodecyl-ammoniumchlorid.

Im Unterschied zu Beispiel 10, in dem die Länge aller Alkylketten im gleichen Maß zunahm, wurde im vorliegenden Beispiel nur eine Kette (von Methyl auf Dodecyl) verlängert.

| Alkyl(aryl) ammoniumchlorid Molmasse [g/mol] | EHZ [s] EA / EE | Biegefestigkeit [MPa] Maximalwert Mittelwert | Biegemodul [MPa] Maximalwert Mittelwert |
|---|---|---|---|
| Benzyl-**tri-methyl** | 63 / 216 | Xₘₐₓ = 47 | Xₘₐₓ = 1321 |
| 185 | 62 / 207 | ∅ = 44 ± 3 | ∅ = 1200 ± 100 |
| Benzyl-**di-methyl-** | 87 / 222 | Xₘₐₓ = 77 | Xₘₐₓ = 2519 |
| **dodecyl** 340 | 86 / 222 | ∅ = 72 ± 4 | ∅ = 2189 ± 168 |

Wie sich aus den Tabellenwerten ergibt, verlängert sich die Erhärtungszeit, vorrangig über den Erhärtungsbeginn, bei Verlängerung einer Alkylkette von Methyl auf Dodecyl. Das Erhärtungsende bleibt im Wesentlichen konstant, was zu einer Snap-Set Verbesserung führt. Biegefestigkeit und Biegemodul steigen deutlich an.

### Beispiel 12

Um den Einfluss einer aromatischen Gruppe im Coinitiator auf die Aushärtungscharakteristik und die mechanischen Eigenschaften (Biegemodul und Biegefestigkeit) näher zu untersuchen, wurden vergleichende Versuche von Tetraethylammoniumchlorid (Ethyl-tri-ethyl-ammoniumchlorid) mit Benzyl-trimethyl-ammoniumchlorid und von Tetrabutylammoniumchlorid (Butyl-tri-butyl-ammoniumchlorid) mit Benzyl-tributyl-ammoniumchlorid durchgeführt. Die Ergebnisse der Versuche sind in der nachfolgenden Tabelle zusammengefasst.

| Alkyl(aryl) ammoniumchlorid Molmasse [g/mol] | EHZ [s] EA / EE | Biegefestigkeit [MPa] Maximalwert Mittelwert | Biegemodul [MPa] Maximalwert Mittelwert |
|---|---|---|---|
| **Ethyl**-tri- | 63 / 178 | Xₘₐₓ = 77 | Xₘₐₓ = 2377 |
| ethyl 166 | 63 / 174 | ∅ = 71 ± 6 | ∅ = 2135 ± 182 |
| **Benzyl**-tri- | 118 / 336 | Xₘₐₓ = 58 | Xₘₐₓ = 1452 |
| ethyl 227 | | ∅ = 52 ± 4 | ∅ = 1280 ± 95 |
| **Butyl**-tri- | 105 / 228 | Xₘₐₓ = 78 | Xₘₐₓ = 2809 |
| butyl 278 | 114 / 226 | ∅ = 71 ± 5 | ∅ = 2306 ± 201 |
| **Benzyl**-tri- | 147 / 324 | Xₘₐₓ = 78 | Xₘₐₓ = 2174 |
| butyl 311 | 151 / 345 | ∅ = 69 ± 6 | ∅ = 2002 ± 118 |

Wie sich aus den Tabellenwerten ergibt, führt der Austausch einer aliphatischen (Ethyl- bzw. Butyl-) Gruppe gegen eine aromatische (Benzyl-) Gruppe zu einer Verlängerung der Erhärtungszeiten (Erhärtungsbeginn und -ende) und zu einer deutlichen Verschlechterung des Snap-Set. Die Biegefestigkeit und die Biegemoduli sinken ebenfalls, wobei diese Effekte mit steigender Kettenlänge abnehmen.

### Beispiel 13

Um den Einfluss unterschiedlicher Substituenten an einem aliphatischen Coinitiatorgrundmolekül zu untersuchen, wurden für das Tetraalkylammoniumchlorid die folgenden Substituenten ausgewählt:

| | |
|---|---|
| aromatischer Substituent | = Benzyl |
| kurzer aliphatischer Substituent | = Methyl |
| langer aliphatischer Substituent | = Dodecyl |
| Substituent mit OH-Gruppe | = 2-Hydroxy-ethyl |

Der Einfluss der unterschiedlichen Substituenten auf die Erhärtungszeiten, Biegefestigkeiten und Biegemoduli der Alkyl(aryl)ammoniumchloride ist in der nachfolgenden Tabelle zusammengefasst.

| Alkyl (aryl) ammoniumchlorid Molmasse [g/mol] | EHZ [s] EA / EE | Biegefestigkeit [MPa] Maximalwert Mittelwert | Biegemodul [MPa] Maximalwert Mittelwert |
|---|---|---|---|
| **Benzyl**-dimethyl- | 87 / 222 | Xₘₐₓ = 77 | Xₘₐₓ = 2519 |
| dodecyl 340 | 86 / 222 | ∅ = 72 ± 4 | ∅ = 2189 ± 168 |
| **Methyl**-dimethyl- | 75 / 318 | Xₘₐₓ = 71 | Xₘₐₓ = 2148 |
| dodecyl 264 | 69 / 318 | ∅ = 65 ± 4 | ∅ = 1852 ± 140 |
| **Dodecyl**-dimethyl- | 255/>1800 | Xₘₐₓ = 35 | Xₘₐₓ = 875 |
| dodecyl 418 | 288/>1800 | ∅ = 31 ± 2 | ∅ = 736 ± 58 |
| **2-Hydroxyethyl-** | 110 / 315 | Xₘₐₓ = 71 | Xₘₐₓ = 2221 |
| dimethyl-dodecyl 216 | 93 / 288 | ∅ = 66 ± 4 | ∅ = 2014 ± 143 |

Aus den Tabellenwerten ergibt sich, dass:
- bei Verwendung von Trimethyl-dodecyl-ammoniumchlorid (Methyl-dimethyl-dodecyl-ammoniumchlorid) als Coinitiator ein schneller Erhärtungsbeginn und ein langsames Erhärtungsende einstellt und das Material eine Biegefestigkeit von etwa 70 MPa und ein Biegemodul von etwa 2100 MPa aufweist;
- bei Austausch einer Methylgruppe durch eine weitere Dodecylgruppe (Dodecyl-dimethyl-dodecyl-ammoniumchlorid) sich Erhärtungsbeginn und -ende verlangsamen, so dass selbst nach 30 Minuten noch keine befriedigende Aushärtung erreicht ist. Ebenfalls fallen Biegefestigkeit und Biegemodul stark ab, so dass ein derart langkettiges Alkylammoniumsalz für den Einsatz in polymerisierbaren Dentalmaterialien als Coinitiator für das Initiatorsystem gemäß dem Oberbegriff von Anspruch 1 ungeeignet ist.
- der Austausch einer Methylgruppe gegen eine Benzylgruppe zu kürzeren Erhärtungszeiten und einem deutlich verbesserten Snap-Set, sowie zu besseren mechanischen Eigenschaften, d.h. höheren Biegefestigkeits-und Biegemodulwerten, führt.

### Beispiel 14

In vorliegendem Beispiel wurde der Einfluss des Gehaltes an erfindungsgemäßen Coinitiator auf die Reaktivität und die mechanischen Eigenschaften des Dentalmaterials untersucht.

Die dabei verwendeten unterschiedlichen Mengen des Coinitiators Benzyl-tributyl-ammoniumchlorid und deren Einfluss auf die EHZ (EA, EE) Biegefestigkeit und Biegemoduli sind in der nachfolgenden Tabelle zusammengefasst.

| Benzyl-tributyl-ammoniumchlorid Gehalt bezogen auf Basispaste [mmol] [Gew.-%] | EHZ [s] EA / EE | Biegefestigkeit [MPa] Maximalwert Mittelwert | Biegemodul [MPa] Maximalwert Mittelwert |
|---|---|---|---|
| 1,9654 | 159 / 301 | Xₘₐₓ = 60 | Xₘₐₓ = 1760 |
| 0,6122 | 141 / 300 | ∅ = 58 ± 2 | ∅ = 1545 ± 119 |
| 3,9307 | 141 / 315 | Xₘₐₓ = 81 | Xₘₐₓ = 2479 |
| 1,2244 | 147 / 312 | ∅ = 75 ± 7 | ∅ = 2291 ± 175 |
| 7,8613 | 169 / 375 | Xₘₐₓ = 73 | Xₘₐₓ = 2329 |
| 2,4488 | 165 / 348 | ∅ = 70 ± 3 | ∅ = 2158 ± 96 |
| 15,7226 | 147 / 324 | Xₘₐₓ = 78 | Xₘₐₓ = 2174 |
| 4,8976 | 151 / 345 | ∅ = 69 ± 6 | ∅ = 2002 ± 118 |
| 31,4452 | 69 / 228 | Xₘₐₓ = 75 | Xₘₐₓ = 2314 |
| 9,7952 | 69 / 219 | ∅ = 70 ± 5 | ∅ = 2217 ± 134 |

Aus den Tabellenwerten ergibt sich, dass der Coinitiator über den gesamten untersuchten Konzentrationsbereich gute Ergebnisse in Bezug auf die EHZ (EA, EE) Biegefestigkeit und Biegemodul erzielt, wobei die besten Ergebnisse mit etwa 1,2 Gew.-% erreicht wurden.

### Beispiel 15

In vorliegendem Beispiel wurde der Einfluss des Halogenions des Coinitiators auf die Reaktivität und die mechanischen Eigenschaften des Dentalmaterials untersucht. Da nach den eingangs erwähnten Untersuchungen von Bredereck et *al*. das Halogenion direkt am radikalischen Härtemechanismus beteiligt sein soll ergibt sich die Frage, ob die Art des Halogenions (Fluorid, Chlorid, Bromid oder Iodid) ebenfalls Einfluß auf die Reaktivität und mechanischen Eigenschaften entsprechender Dentalmaterialien hat. Die experimentelle Untersuchung dieser Frage wurde anhand von Tetraethylammoniumhalogenverbindungen durchgeführt. Dazu wurde die Molzahl zugesetzter Tetraethylammoniumsalze und damit auch die Molzahl Halogenid-Ionen konstant gehalten. Die Ergebnisse sind in der nachfolgenden Tabelle zusammengefasst.

| Tetraethyl-ammoniumsalz Mol- masse [g/mol] | EHZ [s] EA / EE | Biegefestigkeit [MPa] Maximalwert Mittelwert | Biegemodul [MPa] Maximalwert Mittelwert |
|---|---|---|---|
| Fluorid*2H₂O 185 | keine Aushärtung | | |
| Chlorid | 63 / 178 | Xₘₐₓ = 77 | Xₘₐₓ = 2377 |
| 166 | 63 / 174 | ∅ = 71 ± 6 | ∅ = 2135 ± 182 |
| Bromid | 42 / 216 | Xₘₐₓ = 63 | Xₘₐₓ = 2255 |
| 210 | 45 / 216 | ∅ = 55 ± 6 | ∅ = 1939 ± 168 |

Aus den Tabellenwerten ergibt sich, dass die untersuchten Halogenionen auf unterschiedliche Weise die untersuchten Parameter beeinflussen. Für das Fluorid(Dihydrate) wurde keine Aushärtung (bis 30 Minuten) festgestellt. Bei dem Übergang vom Chlorid zum Bromid treten eine beschleunigte Anfangserhärtung und eine verzögerte Endhärtung auf. Unter den untersuchten Halogenionen zeichnet sich das Chlorid durch bestes Snap Set, Biegefestigkeit und Biegemodul aus.

## Patentansprüche

1. Polymerisierbares Dentalmaterial aus wenigstens zwei Komponenten, **dadurch gekennzeichnet, dass** es die folgenden Initiatorkomponenten umfasst:
Komponente 1 enthaltend
a) das Salz einer CH-aciden Verbindung, wobei die CHacide Verbindung eine radikalische Polymerisation auslösen kann,
Komponente 2 enthaltend
b) eine Säure, deren Säurestärke größer ist als die der in der in Komponente 1 als Salz vorliegenden CH-aciden Verbindung,
wobei mindestens eine der Komponenten des polymerisierbaren Dentalmaterials radikalisch polymerisierbare Monomere enthält und
mindestens eine der Komponenten eine Coinitiatorverbindung der Formel 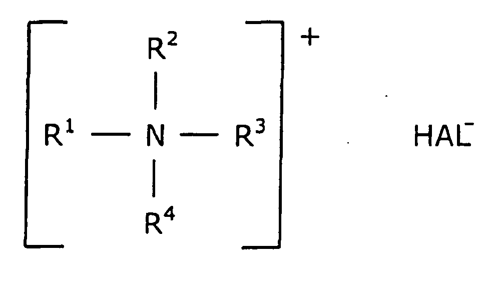
enthält, mit
HAL⁻ = Cl⁻, I⁻ oder Br⁻ und
R¹, R², R³ und R⁴ = lineares oder verzweigtes Alkyl mit 1 bis 12 C-Atomen, vorzugsweise 2 bis 6 C-Atomen,
oder
R¹, R² und R³ = lineares oder verzweigtes Alkyl mit 1 bis 12 C-Atomen, vorzugsweise 2 bis 6 C-Atomen und R⁴ = Benzyl,
wobei höchstens eine R-Gruppe eine C₁₂-Alkylgruppe ist,
wobei die Coinitiatorverbindung nicht Lauryltrimethylammoniumchlorid oder Lauryldimethylbenzylammoniumchlorid ist.

2. Polymerisierbares Dentalmaterial nach Anspruch 1, **dadurch gekennzeichnet, dass** die Coinitiatorverbindung Benzyl-trimethyl-ammoniumchlorid ist.

3. Polymerisierbares Dentalmaterial nach Anspruch 1, **dadurch gekennzeichnet, dass** die Coinitiatorverbindung Benzyl-tributyl-ammoniumchlorid ist.

4. Polymerisierbares Dentalmaterial nach Anspruch 1, **dadurch gekennzeichnet, dass** die Menge der Coinitiatorverbindung bezogen auf das Dentalmaterial 0,3 - 5 Gew.-%, vorzugsweise zwischen 0,3 und 2,5 Gew.-%, besonders bevorzugt zwischen 0,3 und 0,65 Gew.-% beträgt oder die Menge des Coinitiators bezogen auf eine der Komponenten 1 oder 2 bei einer 1:1 Anmischung beider Komponenten 0,6 - 10 Gew.-%, vorzugsweise zwischen 0,6 und 5 Gew.-%, besonders bevorzugt zwischen 0,6 und 1,3 Gew.-% beträgt.

5. Polymerisierbares Dentalmaterial nach einem der vorangehenden Ansprüche, **dadurch gekennzeichnet, dass** die Coinitiatorverbindung in der Komponente 1 und/oder der Komponente 2, vorzugsweise in der Komponente 1 vorliegt.

6. Polymerisierbares Dentalmaterial nach einem der vorangehenden Ansprüche, **dadurch gekennzeichnet, dass** das polymerisierbare Dentalmaterial insgesamt über 50 Gew.-%, vorzugsweise über 60 Gew.-%, weiter vorzugsweise über 70 Gew.-%, weiter vorzugsweise über 80 Gew.-%, weiter vorzugsweise über 90 Gew.-%, weiter vorzugsweise über 95 Gew.-%, weiter vorzugsweise über 98 Gew.-% radikalisch polymerisierbare Monomere enthält.

7. Polymerisierbares Dentalmaterial nach einem der vorangehenden Ansprüche, **dadurch gekennzeichnet, dass** die Komponente 1 über 50 Gew.-%, vorzugsweise über 60 Gew.-%, weiter vorzugsweise über 70 Gew.-%, weiter vorzugsweise über 80 Gew.-%, weiter vorzugsweise über 90 Gew.-%, weiter vorzugsweise über 95 Gew.-%, weiter vorzugsweise über 98 Gew.-% radikalisch polymerisierbare Monomere enthält.

8. Polymerisierbares Dentalmaterial nach einem der vorangehenden Ansprüche, **dadurch gekennzeichnet, dass** die Komponente 1 und die Komponente 2 radikalisch polymerisierbare Monomere enthalten.

9. Polymerisierbares Dentalmaterial nach Anspruch 8, **dadurch gekennzeichnet, dass** die Monomere in Komponente 1 mit den Monomeren in Komponente 2 bei der Mischung beider Komponenten radikalisch polymerisieren können.

10. Polymerisierbares Dentalmaterial nach einem der vorangehenden Ansprüche, **dadurch gekennzeichnet, dass** die Komponente 1 in einer ersten Paste und die Komponente 2 in einer zweiten Paste enthalten ist und dass das vorgesehene Mischungsverhältnis 1:10 oder größer, vorzugsweise 1:4 oder größer, weiter vorzugsweise 1:2 oder größer, besonders bevorzugt 1:1 beträgt.

11. Polymerisierbares Dentalmaterial nach Anspruch 10, **dadurch gekennzeichnet, dass** die Pasten mehr als 3 Monate, vorzugsweise mehr als 6 Monate, besonders bevorzugt mehr als 24 Monate farb- und/oder lagerstabil sind.

12. Polymerisierbares Dentalmaterial nach einem der vorangehenden Ansprüche, **dadurch gekennzeichnet, dass** das Salz der CH-aciden Verbindung der Komponente 1 ein Salz der α-Benzoyl-proprionitrile, α-Cyancarbonsäureester und -amide, cyclischer β-Oxonitrile, β-Diketone, cyclischer β-Diketone, cyclischer β-Oxocarbonsäureester, cyclischer β-Oxo-lactone, Malonsäure, Malonsäurederivate, Pyrazolderivate, Barbitursäure oder Barbitursäurederivate ist.

13. Polymerisierbares Dentalmaterial nach einem der vorangehenden Ansprüche, **dadurch gekennzeichnet, dass** das Salz der CH-aciden Verbindung ein Salz ausgewählt aus der Gruppe bestehend aus einwertigen und zweiwertigen Salzen der Alkali- und Erdalkaliionen, vorzugsweise ein Natriumsalz ist.

14. Polymerisierbares Dentalmaterial nach einem der vorangehenden Ansprüche, **dadurch gekennzeichnet, dass** die Säure der Komponente 2 eine anorganische Säure, vorzugsweise Phosphorsäure und/oder Salzsäure ist.

15. Polymerisierbares Dentalmaterial nach einem der vorangehenden Ansprüche, **dadurch gekennzeichnet, dass** die Säure eine organische Säure ist.

16. Polymerisierbares Dentalmaterial nach Anspruch 15, **dadurch gekennzeichnet, dass** die organische Säure eine Monocarbonsäure, ausgewählt aus der Gruppe bestehend aus Ameisensäure, Essigsäure und Benzoesäure bzw. Derivate dieser Säuren oder eine Dicarbonsäure, ausgewählt aus der Gruppe bestehend aus Oxalsäure, Malonsäure, Succinsäure, Adipinsäure, Pimelinsäure, Azelainsäure, Sebacinsäure, Maleinsäure, Fumarsäure, Sorbinsäure, Phthalsäure und Terephthalsäure bzw. Derivate dieser Säuren oder eine Tricarbonsäure, ausgewählt aus der Gruppe bestehend aus Hemimellithsäure, Trimellithsäure, Trimesinsäure, Agaricinsäure, Citronensäure, 1,2,3-Propantricarbonsäure bzw. Derivate dieser Säuren oder eine Multicarbonsäure, ausgewählt aus der Gruppe bestehend aus Pyromellithsäure und Mellithsäure bzw. Derivate dieser Säuren oder eine Polycarbonsäure, ausgewählt aus der Gruppe bestehend aus Polyacrylsäure und Polymethacrylsäure bzw. Derivate dieser Säuren ist.

17. Polymerisierbares Dentalmaterial nach einem der vorangehenden Ansprüche, **dadurch gekennzeichnet, dass** die radikalisch polymerisierbaren Monomere ausgewählt sind aus der Gruppe bestehend aus Acrylsäureester und Methacrylsäureester.

18. Polymerisierbares Dentalmaterial nach einem der vorangehenden Ansprüche, **dadurch gekennzeichnet, dass** mindestens einer der Komponenten des polymerisierbaren Dentalmaterials, vorzugsweise Komponente 1, Übergangsmetallkationen, vorzugsweise Cu²⁺-Ionen, und zur Radikalbildung geeignete Anionen, vorzugsweise Halogenidionen, weiter vorzugsweise Chlorid-Ionen, enthält.

19. Polymerisierbares Dentalmaterial nach einem der vorangehenden Ansprüche, **dadurch gekennzeichnet, dass** mindestens eine der Komponenten des polymerisierbaren Dentalmaterials ein Füllstoff ist, ausgewählt aus der Gruppe bestehend aus micro- und/oder nanoskaligen Füllstoffen, vorzugsweise Metall-, Halbmetall- oder Mischmetalloxide, Silikaten, Nitriden, Sulfaten, Titanaten, Zirkonaten, Stannaten, Wolframaten, Siliciumdioxiden oder einer Mischung aus diesen Verbindungen oder sphärischen Füllstoffen, Quarzpulvern, Glaspulvern, Glaskeramikpulvern oder eine Mischung aus diesen Pulvern oder gefüllten oder ungefüllten Splitterpolymerisaten und/oder Perlpolymerisaten.

20. Polymerisierbares Dentalmaterial nach Anspruch 19, **dadurch gekennzeichnet, dass** der Füllstoff ein oberflächenmodifizierter Füllstoff, vorzugsweise eine organisch oberflächenmodifizierter Füllstoff ist.

21. Polymerisierbares Dentalmaterial nach Anspruch 20, **dadurch gekennzeichnet, dass** der oberflächenmodifizierte Füllstoff auf seiner Oberfläche funktionelle Gruppen besitzt, die mit den Monomeren chemisch, vorzugsweise radikalisch, reagieren können oder eine hohe Affinität zu der aus den Monomeren gebildeten Polymermatrix haben.

22. Polymerisierbares Dentalmaterial nach Anspruch 21, **dadurch gekennzeichnet, dass** der Füllstoff mit reaktiven Acrylat- öder Methacrylatgruppen tragendem Silan oberflächenmodifiziert ist.

23. Verwendung des polymerisierbaren Dentalmaterials nach einem der vorangehenden Ansprüche als Füllungsmaterial, Stumpfaufbaumaterial, Befestigungsmaterial, Bondingmaterial, provisorisches und permanentes Kronen-und Brückenmaterial, zahntechnischer Werkstoff zur Herstellung von Inlays, Onlays, Verblendschalen, künstlichen Zähnen, Modellmaterialien und Fissuren- bzw. Wurzelkanal-Versiegelungsmaterial.

24. Gehärtetes Dentalmaterial, erhältlich aus einem polymerisierbaren Dentalmaterial nach einem der Ansprüche 1 bis 22.

25. Verwendung einer Verbindung der Formel 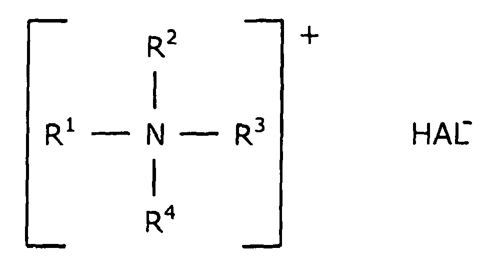 mit
HAL⁻ = Cl⁻, I⁻ oder Br⁻ und
R¹, R², R³ und R⁴ = lineares oder verzweigtes Alkyl mit 1 bis 12 C-Atomen, vorzugsweise 2 bis 6 C-Atomen,
oder
R¹, R² und R³ = lineares oder verzweigtes Alkyl mit 1 bis 12 C-Atomen, vorzugsweise 2 bis 6 C-Atomen und R⁴ = Benzyl,
wobei höchstens eine R-Gruppe eine C₁₂-Alkylgruppe ist,
als Coinitiator in radikalisch polymerisierbaren Dentalmaterialien umfassend die folgenden Initiatorkomponenten:
Komponente 1 enthaltend
a) das Salz einer CH-aciden Verbindung, wobei die CHacide Verbindung eine radikalische Polymerisation auslösen kann,
Komponente 2 enthaltend
b) eine Säure, deren Säurestärke größer ist als die der in der in Komponente 1 als Salz vorliegenden CH-aciden Verbindung,
wobei mindestens eine der Komponenten des polymerisierbaren Dentalmaterials radikalisch polymerisierbare Monomere enthält.

## Claims

1. A polymerisable dental material prepared from at least two components, **characterised in that** it comprises the following initiator components:
component 1 containing
a) the salt of a CH-acidic compound, the CH-acidic compound being capable of initiating free-radical polymerisation,
component 2 containing
b) an acid, the acid strength of which is greater than that of the CH-acidic component present as a salt in component 1,
at least one of the components of the polymerisable dental material containing free-radically polymerisable monomers and
at least one of the components containing a coinitiator compound of formula 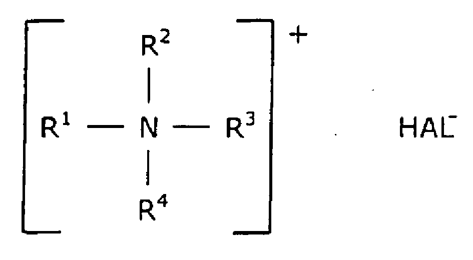
where
HAL⁻ = Cl⁻, I⁻ or Br⁻ and R¹, R², R³ and R⁴ = linear or branched alkyl with 1 to 12 C atoms, preferably 2 to 6 C atoms,
or
R¹, R² and R³ = linear or branched alkyl with 1 to 12 C atoms, preferably 2 to 6 C atoms and R⁴ = benzyl,
at most one R group being a C₁₂ alkyl group,
the coinitiator compound not being lauryltrimethylammonium chloride or lauryldimethylbenzylammonium chloride.

2. A polymerisable dental material according to claim 1, **characterised in that** the coinitiator compound is benzyltrimethylammonium chloride.

3. A polymerisable dental material according to claim 1, **characterised in that** the coinitiator compound is benzyltributylammonium chloride.

4. A polymerisable dental material according to claim 1, **characterised in that** the quantity of coinitiator compound relative to the dental material amounts to 0.3-5 wt.%, preferably between 0.3 and 2.5 wt.%, particularly preferably between 0.3 and 0.65 wt.% or the quantity of coinitiator relative to one of components 1 or 2 in a 1:1 blend of the two components amounts to 0.6-10 wt.%, preferably between 0.6 and 5 wt.%, particularly preferably between 0.6 and 1.3 wt.%.

5. A polymerisable dental material according to any one of the preceding claims, **characterised in that** the coinitiator compound is present in component 1 and/or component 2, preferably in component 1.

6. A polymerisable dental material according to any one of the preceding claims, **characterised in that** the polymerisable dental material contains in total greater than 50 wt.%, preferably greater than 60 wt.%, more preferably greater than 70 wt.%, more preferably greater than 80 wt.%, more preferably greater than 90 wt.%, more preferably greater than 95 wt.%, more preferably greater than 98 wt.% of free-radically polymerisable monomers.

7. A polymerisable dental material according to any one of the preceding claims, **characterised in that** component 1 contains greater than 50 wt.%, preferably greater than 60 wt.%, more preferably greater than 70 wt.%, more preferably greater than 80 wt.%, more preferably greater than 90 wt.%, more preferably greater than 95 wt.%, more preferably greater than 98 wt.% of free-radically polymerisable monomers.

8. A polymerisable dental material according to any one of the preceding claims, **characterised in that** component 1 and component 2 contain free-radically polymerisable monomers.

9. A polymerisable dental material according to claim 8, **characterised in that** the monomers in component 1 are capable of free-radically polymerising with the monomers in component 2 when the two components are mixed.

10. A polymerisable dental material according to any one of the preceding claims, **characterised in that** component 1 is present in a first paste and component 2 in a second paste and **in that** the intended mixing ratio amounts to 1:10 or above, preferably 1:4 or above, more preferably 1:2 or above, particularly preferably 1:1.

11. A polymerisable dental material according to claim 10, **characterised in that** the pastes are colour-stable and/or storage-stable for more than 3 months, preferably more than 6 months, particularly preferably more than 24 months.

12. A polymerisable dental material according to any one of the preceding claims, **characterised in that** the salt of the component 1 CH-acidic compound is a salt of α-benzoylpropionitrile, α-cyanocarboxylic acid esters and amides, cyclic β-oxonitriles, β-diketones, cyclic β-diketones, cyclic β-oxocarboxylic acid esters, cyclic β-oxolactones, malonic acid, malonic acid derivatives, pyrazole derivatives, barbituric acid or barbituric acid derivatives.

13. A polymerisable dental material according to any one of the preceding claims, **characterised in that** the salt of the CH-acidic compound is a salt selected from the group consisting of monovalent and divalent salts of alkali metal and alkaline earth metal ions, preferably a sodium salt.

14. A polymerisable dental material according to any one of the preceding claims, **characterised in that** the component 2 acid is an inorganic acid, preferably phosphoric acid and/or hydrochloric acid.

15. A polymerisable dental material according to any one of the preceding claims, **characterised in that** the acid is an organic acid.

16. A polymerisable dental material according to claim 15, **characterised in that** the organic acid is a monocarboxylic acid selected from the group consisting of formic acid, acetic acid and benzoic acid or derivatives of these acids or a dicarboxylic acid selected from the group consisting of oxalic acid, malonic acid, succinic acid, adipic acid, pimelic acid, azelaic acid, sebacic acid, maleic acid, fumaric acid, sorbic acid, phthalic acid and terephthalic acid or derivatives of these acids or a tricarboxylic acid selected from the group consisting of hemimellitic acid, trimellitic acid, trimesic acid, agaricinic acid, citric acid, 1,2,3-propanetricarboxylic acid or derivatives of these acids or a multicarboxylic acid selected from the group consisting of pyromellitic acid and mellitic acid or derivatives of these acids or a polycarboxylic acid selected from the group consisting of polyacrylic acid and polymethacrylic acid or derivatives of these acids.

17. A polymerisable dental material according to any one of the preceding claims, **characterised in that** the free-radically polymerisable monomers are selected from the group consisting of acrylic acid esters and methacrylic acid esters.

18. A polymerisable dental material according to any one of the preceding claims, **characterised in that** at least one of the components of the polymerisable dental material, preferably component 1, contains transition metal cations, preferably Cu²⁺ ions, and anions suitable for forming free-radicals, preferably halide ions, more preferably chloride ions.

19. A polymerisable dental material according to any one of the preceding claims, **characterised in that** at least one of the components of the polymerisable dental material is a filler selected from the group consisting of micro- and/or nanoscale fillers, preferably metal, semimetal or mischmetal oxides, silicates, nitrides, sulfates, titanates, zirconates, stannates, tungstates, silicon dioxides or a mixture of these compounds or spherical fillers, quartz powders, glass powders, glass-ceramic powders or a mixture of these powders or filled or unfilled splinter polymers and/or bead polymers.

20. A polymerisable dental material according to claim 19, **characterised in that** the filler is a surface-modified filler, preferably an organically surface-modified filler.

21. A polymerisable dental material according to claim 20, **characterised in that** the surface-modified filler has on its surface functional groups which are capable of reacting chemically, preferably free-radically, with the monomers or have an elevated affinity for the polymer matrix formed from the monomers.

22. A polymerisable dental material according to claim 21, **characterised in that** the filler is surface-modified with silane bearing reactive acrylate or methacrylate groups.

23. Use of the polymerisable dental material according to any one of the preceding claims as a filling material, core build-up material, cement, bonding material, provisional and permanent crown and bridge material, dental technology material for producing inlays, onlays, veneers, artificial teeth, modelling materials and fissure or root canal sealant.

24. A cured dental material obtainable from a polymerisable dental material according to any one of claims 1 to 22.

25. Use of a compound of the formula 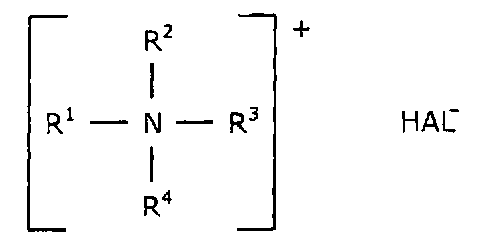 where
HAL⁻ = Cl⁻, I⁻ or Br⁻ and
R¹, R², R³ and R⁴ = linear or branched alkyl with 1 to 12 C atoms, preferably 2 to 6 C atoms,
or
R¹, R² and R³ = linear or branched alkyl with 1 to 12 C atoms, preferably 2 to 6 C atoms and R⁴ = benzyl,
at most one R group being a C₁₂ alkyl group,
as coinitiator in free-radically polymerisable dental materials comprising the following initiator components:
component 1 containing
a) the salt of a CH-acidic compound, the CH-acidic compound being capable of initiating free-radical polymerisation.
component 2 containing
b) an acid, the acid strength of which is greater than that of the CH-acidic component present as a salt in component 1,
at least one of the components of the polymerisable dental material containing free-radically polymerisable monomers.

## Revendications

1. Matériau dentaire polymérisable en au moins deux composants, **caractérisé en ce qu'**il comprend les composants excitateurs suivants :
composant 1 contenant
a) le sel d'un composé à CH acide, le composé à CH acide pouvant déclencher une polymérisation radicalaire, composant 2, contenant
b) un acide dont la force est plus grande que celle du composé à CH acide présent comme sel dans le composant 1, sachant qu'au moins un des composants du matériau dentaire polymérisable contient des monomères polymérisables radicalairement et
qu'au moins un des composants contient un composé coexcitateur de la formule 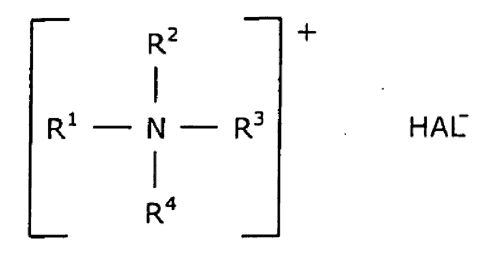
avec
HAL⁻ = Cl⁻, I⁻ ou Br⁻ et
R¹, R², R³ et R⁴ = alkyles linéaires ou ramifiés avec1 à 12 atomes de C, de préférence 2 à 6 atomes de C,
ou
R¹, R² et R³ = alkyles linéaires ou ramifiés avec 1 à 12 atomes de C, de préférence 2 à 6 atomes de C et R⁴ = benzyle,
sachant qu'au plus un groupe R est un groupe alkyle en C₁₂,
sachant que le composé coexcitateur n'est pas du chlorure de lauryltriméthylammonium ni du lauryldiméthylbenzylammonium.

2. Matériau dentaire polymérisable selon la revendication 1, **caractérisé en ce que** le composé coexcitateur est du chlorure de benzyl-triméthyl-ammonium.

3. Matériau dentaire polymérisable selon la revendication 1, **caractérisé en ce que** le composé coexcitateur est du chlorure de benzyl-tributyl-ammonium.

4. Matériau dentaire polymérisable selon la revendication 1, **caractérisé en ce que** la quantité du composé coexcitateur, rapportée au matériau dentaire, est de 0,3 à 5 % en poids, de préférence entre 0,3 et 2,5 % en poids, encore plus préférablement entre 0,3 et 0,65 % en poids, ou que la quantité du coexcitateur, rapportée à un des composés 1 ou 2 pour un mélangeage 1 : 1 des deux composants est de 0,6 à 10 % en poids, de préférence entre 0,6 et 5 % en poids, encore plus préférablement entre 0,6 et 1,3 % en poids.

5. Matériau dentaire polymérisable selon l'une quelconque des revendications précédentes, **caractérisé en ce que** le composé coexcitateur est présent dans le composant 1 et/ou dans le composant 2, de préférence dans le composant 1.

6. Matériau dentaire polymérisable selon l'une quelconque des revendications précédentes, **caractérisé en ce que** le matériau dentaire polymérisable contient en tout plus de 50 % en poids, de préférence plus de 60 % en poids, encore plus préférablement plus de 70 % en poids, encore plus préférablement plus de 80 % en poids, encore plus préférablement plus de 90 % en poids, encore plus préférablement plus de 95 % en poids, encore plus préférablement plus de 98 % en poids de monomères polymérisables radicalairement.

7. Matériau dentaire polymérisable selon l'une quelconque des revendications précédentes, **caractérisé en ce que** le composant 1 contient en tout plus de 50 % en poids, de préférence plus de 60 % en poids, encore plus préférablement plus de 70 % en poids, encore plus préférablement plus de 80 % en poids, encore plus préférablement plus de 90 % en poids, encore plus préférablement plus de 95 % en poids, encore plus préférablement plus de 98 % en poids de monomères polymérisables radicalairement.

8. Matériau dentaire polymérisable selon l'une quelconque des revendications précédentes, **caractérisé en ce que** le composant 1 et le composant 2 contiennent des monomères polymérisables radicalairement.

9. Matériau dentaire polymérisable selon la revendication 8, **caractérisé en ce que**, lors du mélangeage des deux composants, les monomères dans le composant 1 peuvent polymériser radicalairement avec les monomères dans le composant 2.

10. Matériau dentaire polymérisable selon l'une quelconque des revendications précédentes, **caractérisé en ce que** le composant 1 est contenu dans une première pâte et que le composant 2 est contenu dans un autre pâte, et **en ce que** le rapport de mélange prévu est de 1 : 10 ou plus, de préférence de 1 : 4 ou plus, encore plus préférablement de 1 : 2 ou plus, de façon particulièrement préférée de 1 : 1.

11. Matériau dentaire polymérisable selon la revendication 10, **caractérisé en ce que** les pâtes sont stables en couleur et/ou au stockage plus de 3 mois, de préférence plus de 6 mois, de façon particulièrement préférée plus de 24 mois.

12. Matériau dentaire polymérisable selon l'une quelconque des revendications précédentes, **caractérisé en ce que** le sel du composé à acidité CH du composant 1 est un sel de α-benzoyl-proprionitriles, d'esters et d'amides d'acides α-cyanocarboxyliques, de β-oxonitriles cycliques, de ß-dicétones, de ß-dicétones cycliques, d'esters d'acides ß-oxocarboxyliques cycliques, de ß-oxo-lactones cycliques, d'acide malonique, de dérivés d'acide malonique, de dérivés du pyrazole, d'acides barbituriques ou de dérivés d'acides barbituriques.

13. Matériau dentaire polymérisable selon l'une quelconque des revendications précédentes, **caractérisé en ce que** le sel du composé à acidité CH est un sel choisi parmi le groupe constitué de sels monovalents et divalents d'ions alcalin et alcalino-terreux, de préférence un sel de sodium.

14. Matériau dentaire polymérisable selon l'une quelconque des revendications précédentes, **caractérisé en ce que** l'acide du composant 2 est un acide inorganique, de préférence de l'acide phosphorique et/ou de l'acide chlorhydrique.

15. Matériau dentaire polymérisable selon l'une quelconque des revendications précédentes, **caractérisé en ce que** l'acide est un acide organique.

16. Matériau dentaire polymérisable selon la revendication 15, **caractérisé en ce que** l'acide organique est un acide monocarboxylique, choisi parmi le groupe constitué de l'acide formique, de l'acide acétique et de l'acide benzoïque, respectivement de dérivés de ces acides ou un acide dicarboxylique, choisi parmi le groupe constitué de l'acide oxalique, de l'acide malonique, de l'acide succinique, de l'acide adipique, de l'acide pimélique, de l'acide azélaïque, de l'acide sébacique, de l'acide maléique, de l'acide fumarique, de l'acide sorbique, de l'acide phtalique et de l'acide téréphtalique, respectivement de dérivés de ces acides ou d'un acide tricarboxylique, choisi parmi le groupe constitué de l'acide hémimellithique, de l'acide trimellithique, de l'acide trimésique, de l'acide agaricique, de l'acide citrique, de l'acide 1,2,3-propane tricarboxylique, respectivement de dérivés de ces acides, d'un acide multicarboxylique, choisi parmi le groupe constitué de l'acide pyromellithique et de l'acide mellithique, respectivement de dérivés de ces acides, ou d'un acide polycarboxylique, choisi parmi le groupe constitué de l'acide polyacrylique et de l'acide polyméthacrylique, respectivement de dérivés de ces acides.

17. Matériau dentaire polymérisable selon l'une quelconque des revendications précédentes, **caractérisé en ce que** les monomères polymérisables radicalairement sont choisis parmi le groupe constitué des esters de l'acide acrylique et de l'acide méthacrylique.

18. Matériau dentaire polymérisable selon l'une quelconque des revendications précédentes, **caractérisé en ce qu'**au moins un des composants du matériau dentaire polymérisable, de préférence le composant 1, contient des cations de métaux de transition, de préférence des ions Cu²⁺, et des anions appropriés à la formation de radicaux, de préférence des ions halogénure, plus préférablement des ions chlorure.

19. Matériau dentaire polymérisable selon l'une quelconque des revendications précédentes, **caractérisé en ce qu'**au moins un des composants du matériau dentaire polymérisable est une charge, choisie parmi le groupe constitué de charges à l'échelle micro et/ou nano, de préférence des oxydes de métaux, de métalloïdes ou de métaux mixtes, des silicates, des nitrures, des sulfates, des titanates, des zirconates, des stannates, des tungstates, des dioxydes de silicium ou un mélange de ces composés, ou des charges sphériques, des poudres de quartz, des poudres de verre, des poudres de céramique de verre, ou un mélange de ces poudres, ou des polymères fragmentés ou perlés remplis ou non remplis.

20. Matériau dentaire polymérisable selon la revendication 19, **caractérisé en ce que** la charge est une charge modifiée en surface, de préférence une charge modifiée organiquement en surface.

21. Matériau dentaire polymérisable selon la revendication 20, **caractérisé en ce que** la charge modifiée en surface possède à sa surface des groupes fonctionnels susceptibles de réagir chimiquement, de préférence radicalairement, avec les monomères, ou qui ont une haute affinité pour la matrice de polymère formées en les monomères.

22. Matériau dentaire polymérisable selon la revendication 21, **caractérisé en ce que** la charge est modifiée en surface par un silane porteur de groupes réactifs acrylate ou méthacrylate.

23. Utilisation du matériau dentaire polymérisable selon l'une quelconque des revendications précédentes comme matériau d'obturation, matériau d'élaboration sur moignon, matériau de fixation, matériau de bonding, matériau de couronnes et de bridges provisoires ou permanents, matériau technique dentaire pour la fabrication d'inlays, d'onlays, de placages, de dents artificielles, de matériaux pour modèles, et de matériaux d'obturation de fissures et de canal pulpaire.

24. Matériau dentaire durci, pouvant être obtenu en un matériau dentaire polymérisable selon l'une quelconque des revendications 1 à 22.

25. Utilisation d'un composé de la formule 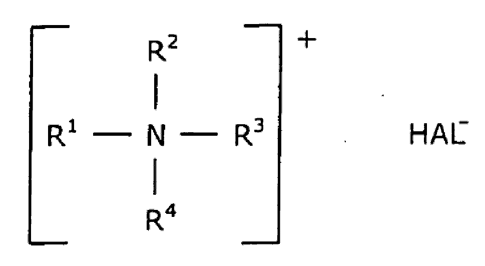 avec
HAL⁻ = Cl⁻, I⁻ ou Br⁻ et
R¹, R², R³ et R⁴ = alkyles linéaires ou ramifiés avec 1 à 12 atomes de C, de préférence 2 à 6 atomes de C,
ou
R¹, R² et R³ = alkyles linéaires ou ramifiés avec 1 à 12 atomes de C, de préférence 2 à 6 atomes de C et R⁴ = benzyle
sachant qu'au plus un groupe R est un groupe alkyle en C₁₂,
comme coexcitateur dans des matériaux dentaires polymérisables radicalairement, comprenant les composants excitateurs suivants :
composant 1 contenant
a) le sel d'un composé à CH acide, le composé à CH acide pouvant déclencher une polymérisation radicalaire, composant 2, contenant
b) un acide dont la force est plus grande que celle du
composé à CH acide présent comme sel dans le composant 1, sachant qu'au moins un des composants du matériau dentaire polymérisable contient des monomères polymérisables radicalairement.
